# EUROPEAN PATENT SPECIFICATION

(11) **EP 0 355 687 B1**
(45) Date of publication and mention of the grant of the patent: **15.03.1995**
(21) Application number: 89115089.8
(22) Date of filing: 16.08.1989
(51) Int. Cl.: G01N 33/543, G01N 33/52

(54) **Self-blocked assay support**
Selbstblockierender Testträger
Support d'essai autobloquant

(30) Priority: 22.08.1988 US 235411
(43) Date of publication of application: 28.02.1990
(73) Proprietor: W.R. Grace & Co.-Conn., New York, New York 10036 (US)
(72) Inventor: Rudolph, Julie Liao, Carlisle Massachusetts (US); Parham, Marc Ellous, Bedford Massachusetts (US)
(74) Representative: UEXKÜLL & STOLBERG

(56) References cited:
- EP-A- 0 172 362
- US-A- 4 435 029
- US-A- 4 442 259
- US-A- 4 794 090

## Description

This is a continuation-in-part of copending U.S. 4,794,090 (Parham and Rudolph), published December 27, 1988.

### BACKGROUND OF THE INVENTION

This invention relates to the field of diagnostics or other analytical testing. Diagnostic and other assays typically utilize a solid support or matrix for a bioaffinity agent, which detects the presence of a particular desired protein (the "target" protein). These bioaffinity agents, usually antibodies to the target protein or antigen, are immobilized in some manner on the matrix to yield an "assay matrix" or support which is used in the diagnostic testing. The bioaffinity agent binds the target protein, fixing it to the assay matrix and making it available for detection via recognition assay techniques. It now has been found that the application of certain polymeric coatings onto solid assay supports (porous or nonporous) provides two advantages of major significance in the performance of assays. First, the polymeric coatings essentially eliminate nonspecific binding of protein onto the support surface by providing a self-blocked surface. Second, these polymeric coatings eliminate the need for immobilizing very costly bioaffinlty agents onto the membrane in assays for antigens which are associated with cell membranes or cell surfaces, such as lipopolysaccharides ("LPS"), for example.

Diagnostic assays most frequently are conducted on membrane supports. Diagnostic membranes are conventionally prepared from microporous membranes, such as nylon, in the following manner. A solution containing a bioaffinity agent is dotted onto the membrane and dried, becoming immobilized on the membrane by passive adsorption. Next, at least one blocking step is required for the prevention of nonspecific binding of the target protein, or a recognition protein for the target protein, or other proteins which may be present in the test sample or reagent solutions. Nonspecific binding across the entire membrane renders the assay inaccurate and unreadable. This is a particular problem when the test sample contains blood or blood proteins. Blocking steps conventionally comprise thoroughly coating the membrane with a protein in order to fill, or block, the nonspecific binding sites otherwise available on the support surface.

The assay typically is conducted by passing test fluid through the membrane. If the target protein is present in the test fluid, it will bind to a unique binding site of the primary antibody (bioaffinity agent) immobilized on the membrane. The membrane then is treated with a recognition conjugate, consisting of a recognition antibody (which binds to a second binding site on the target protein) coupled to an enzyme, such as horseradish peroxidase, which can be induced to generate a color change under positive test conditions. The membrane is rinsed and treated with a substrate for the enzyme-mediated color change reaction. If the target protein was present in the test fluid, it will have become bound to the primary antibody and, in turn, will have bound the recognition conjugate. The enzyme portion of the recognition conjugate will react with the substrate, producing an easily detected color change for a positive assay. If the protein was not present, the recognition conjugate will not be bound to the membranes and no color change will occur upon treatment with the substrate. Supports other than membranes can be used.

It can be seen that the occurrence of nonspecific binding of proteins will seriously impair the usefulness of diagnostic assays of this type. Untreated microporous membranes or other supports display a strong propensity to adsorb proteins on contact. Thus, proteins present in the test fluid would be adsorbed over the whole support, rather than the target protein just being bound to the primary antibody. For example, where the test sample is blood or serum, or where the sample may be contaminated with blood, the blood proteins, including hemoglobin, have a strong propensity for binding to conventionally blocked or untreated membranes. It is preferred to have an assay system in which a positive result is readily and clearly detectable as a sharp colored dot or pattern on a contrasting (white) background.

Even more detrimental to this assay technique is nonspecific adsorption of the recognition conjugate. The utility of the assay depends upon the recognition conjugate binding only to the target protein, which has been bound by the immobilized primary antibody. If the recognition conjugate can be adsorbed directly onto the support itself, false positives or blinding of the results by a color change over the whole support surface will occur, rendering the assay useless.

### SUMMARY OF THE INVENTION

A self-blocked protein non-adsorptive assay support is created by treating an assay support matrix with a solution of an aqueous-based polyurethane in a volatile organic solvent to form a polyurethane polymer coating on the support and immobilizing a bioaffinity agent on the coated support. The polymer-treated support may be used in assays without further processing for prevention of the complications of nonspecific protein binding as discussed above. The assay support of this invention also may be used as a passive adsorption support for determining the presence of cell membrane-associated antigens without the need for immobilization of a bioaffinity agent.

This invention is intended to improve reliability of diagnostic or other assays by significantly reducing susceptibility to false results or to blinding of results due to nonspecific protein adsorption. Use of the self-blocked membrane or support of this invention completely eliminates the protein blocking steps required by conventional methods of preparation. It is particularly intended to provide a self-blocked membrane or support resistant to binding of even hard-to-block proteins such as hemoglobin or the like. The assays of this invention retain a white background during the test protocol which is advantageous for clear distinction between positive and negative results. Moreover, it is intended to provide a diagnostic support which resists deterioration of color formation upon exposure to air or moisture.

This invention also is intended to streamline and simplify the manufacturing procedures for preparing assay supports. Used in these assays, the invention altogether eliminates the need for bioaffinity agents, which are relatively expensive elements of the assay. Moreover, the assay shelf life typically is limited by the stability of the bioaffinity agent. It is therefore a purpose of this invention to provide a very stable, long shelf-life assay membrane.

It is a further object to provide an assay system in which a positive result is apparent substantially instantaneously as a visually detectable color change, thereby eliminating the waiting time necessary with conventional assays.

### DETAILED DESCRIPTION OF THE INVENTION

The self-blocked assay membrane or support of this invention is characterized by a coating of a polyurethane polymer which simultaneously resists assay interference by nonspecific binding of protein and assay reagents. In addition, it acts as a passive adsorption assay support which facilitates the immobilization of cell membrane or cell membrane fragments with which a target antigen is associated. The target antigen then can be detected using conventional assay techniques.

### The Support Materials

The assay support of this invention may be in various forms and may be porous or nonporous. The support may be a microporous or nonwoven membrane. Particulate porous or nonporous media suitable for use in diagnostic assays may be used, as may nonporous devices such as microtiter plates. The choices of support material and porosity depend on the format desired for a particular assay.

Microporous membranes or other support materials such as those now utilized for diagnostics and other assays will be suitable for preparing the treated supports of this invention. Nylon membranes are frequently used. Alternatively, membranes of polypropylene, various polyesters, polyvinyl fluoride, polyvinyl chloride, Teflon™ (E. I. DuPont de Nemours & Co.) or cellulose may be used. Other suitable support materials include polystyrene, polycarbonates, polyvinylidene difluoride, polysulfone, polyacrylates and acrylic acids, polyamide, glass and the like. Various cellulosic materials may be used, including carboxymethylated cellulose, DEAE cellulose, cellulose phosphate, cellulose sulfate, nitrocellulose, carboxymethylated nitrocellulose, DEAE nitrocellulose, nitrocellulose phosphate, nitrocellulose sulfate, cellulose acetate/cellulose nitrate and the like.

Alternatively, porous or nonporous particulate support matrices may be used. For example, silica gel and either inorganic or organic beaded matrices would be suitable. For example, agarose is suitable, as is cross-linked dextran such as Sephadex™ dextran (Pharmacia). The particle size will be chosen according to the format in which the assay support matrix will be used. For example, if the matrix will be in a column or packed bed configuration, the particles must be of sufficient size to allow flow of the test fluid and reagent solutions through the bed. One micron beads may be desirable for use in this embodiment. Again, the material chosen must be insoluble in the solvents used in preparing the treated matrix and conducting the assay.

Membranes, pads or the like of woven or nonwoven materials must be of suitable surface area such that the test fluid and any solutes contained therein will wet the surface. Porosity may be selected so that the solutes will or will not pass through the support, as desired. Membranes with pore sizes of about 0.05 or less to about 30.0 to 50.0 microns or greater are typically used. The selected porosity will depend on the device and desired flow rate, as well as whether it is desired to entrap cells and cell fragments or to let them pass through the support.

### The Polymeric Coating Material

The selected membrane or other support matrix is treated with a solution of an aqueous-based polyurethane polymer in a volatile organic solution to provide the membrane with a polyurethane polymeric coating. This coating confers on the membrane or support resistance to nonspecific protein binding. It also confers on the support the ability to entrap, bind or adsorb cell membranes or fragments thereof, together with any antigens associated therewith.

The aqueous-based polyurethane polymer used in preparing the polymeric coating of this invention may be any of the elastomers described in U.S. 4,442,259 (Isgur et al.), the disclosure of which is hereby incorporated herein in its entirety. Isgur et al. urethane compositions are prepared by chain extending an isocyanate-terminated urethane prepolymer with an equivalent excess of water (based on the isocyanate content of the prepolymer) in the presence of a monofunctional isocyanate group blocking agent. The result is an aqueous-based polyurethane polymer with film-forming capabilities. The monofunctional blocking agent can be any compound capable of reacting with and blocking the isocyanate group so as to retard or prevent its reaction with water. Examples include alcohols such as methanol, ethanol, isopropanol and phenol; primary or secondary monoamines such as methylamine, ethylamine and isopropylamine; oximes such as acetone oxime, butanone oxime and cyclohexanone oxime; alkanol amines such as ethanol amine; and ammonia.

The Isgur prepolymers are prepared by the well known method of reacting a polyol with an excess of an aliphatic or aromatic polyisocyanate. Suitable prepolymers include those formed from polyoxyalklene polyols and polyester polyols. Prepolymers prepared from polyoxyalkylene polyols are preferred, and particularly those having a molecular weight of about 200 to about 10,000. The hydroxyl functionality of the polyol and, thus, of the corresponding isocyanate-terminated prepolymer can be from about 2 to 4 and is preferably from about 2 to about 2.5.

Most preferably, the aqueous-based polyurethane polymer will be Darathane™ WB-22 polyurethane elastomer (Grace Specialty Chemicals Co., W. R. Grace & Co.-Conn.). Darathane WB-22 is an aliphatic urethane polymer in an aqueous solution of about 40% solids in ethanol. Where reference is made herein to "Darathane polymer" it is intended to encompass similar aqueous-based polyurethane polymers as described above.

The polymer coating needed to render the treated support self-blocked will depend on the type of support to be used. That is, either a coating of Darathane polymer is used alone, or a dual polymer coating is used which comprises Darathane polymer and a second polyurethane polymer which is resistant to nonspecific binding of protein.

Where the assay support is a very absorptive material, Darathane polymer alone may be used since the support will absorb the Darathane polymer to form a suitable coating. Cellulosic materials are examples of such absorptive support material, and coating cellulose with Darathane polyurethane polymer will be sufficient to achieve the objectives of this invention. Other absorptive support materials include cellulose mixed-esters, cellulose nitrate, cellulose acetate, and the like.

Where a less absorptive, or a nonadsorptive, support (such as nylon) is used, a dual polymer coating comprising Darathane and a second polyurethane polymer must be present on the treated support. The second polymer is applied as a prepolymer solution in order to aid in binding the Darathane polymer to the surface membrane or support. Without intending to be limited by theory, it is Applicants' belief that the prepolymer binds to the support surface (i.e., by reaction with free primary amine groups on a nylon membrane) and that an interpenetrating network is formed of the Darathane polymer and the second polyurethane polymer. The result is the formation of an adhering Darathane polymer coating on the assay support surface. The second polyurethane polymer component is provided by applying a polyethylene-based urethane prepolymer. Suitable polyurethane prepolymers of this type are disclosed in U.S. 4,137,200 (Wood et al.), the disclosure of which is hereby incorporated herein by reference in its entirety. Most preferable is Hypol 6100^{TM} polyurethane prepolymer (Grace Specialty Chemicals Co., W. R. Grace & Co.-Conn.) and prepolymers availabe under the trademark Biopol from Grace Specialty Chemicals Co., W. R. Grace & Co.-Conn.

The polyurethane prepolymers used in the invention for the second polyurethane polymer component may be prepared by capping a polyoxyalkylene polyol with a polyisocyanate compound, such that the capped product (the prepolymer) has a reaction functionality greater than two. Complete end capping of the polyol is desired. The polyols used to prepare the prepolymer may have an average molecular weight in the range of about 200 to about 20,000. It may be preferred to use a prepolymer substantially or exclusively comprised of ethylene oxide units. Polyethylene glycol-based prepolymers are particularly preferred. The polyols should have a hydroxyl functionality of about 2 or greater, preferably from about 2 to about 6. A wide variety of polyether polyols and polyester polyols may be used.

The polyol is end capped with an aliphatic or aromatic polyisocyanate. Suitable polyisocyanates include, but are not limited to toluene-2,4-diisocyanate, toluene-2,6-diisocyanate, isophorone diisocyanate, and the like. The end capping reaction may be by any convenient method or procedure. For example, the reaction may be conducted in an inert moisture-free environment, such as under a nitrogen blanket, at atmospheric pressure and at temperatures in the range of about 0°C to about 120°C. This invention, however, is not limited to use of the prepolymers described in the above-listed patents.

### Preparation of the Self-Blocked Assay Support

There are two basic procedures by which the self-blocked assay support of this invention is prepared. Where a single polymeric component is employed, a one-step process is used. Where a dual polymer coating is employed, the treatment process may be one-step, in which the support simultaneously is treated with both the Darathane polymer and a polyurethane prepolymer, or it may be two-step, in which the support first is treated with the prepolymer and then is treated with the polymer.

In the single polymer embodiment of this invention, the treated support has a Darathane polymeric coating. An untreated membrane or support is contacted with a solution of Darathane polymer in a volatile organic solvent. Any solvent which is nonreactive with the support will be suitable. Acetone and isopropyl alcohol are particularly suitable. Alcohols and chlorinated hydrocarbons may be used. The concentration of polymer in the solution should be about 0.1 to about 20.0 percent, preferably about 0.1 to about 10.0 percent. The support and polymer solution should remain in contact for at least about one minute, preferably about five to ten minutes, and most preferably at least thirty to sixty minutes. Agitation may be preferred to obtain uniform coating, particularly when coating large pieces of membrane or other support material.

It may be desirable to use a surfactant in preparing the polymer solution. Non-ionic surfactants or detergents such as Tween-20™ or Tween-80™ (ICI United States) or Triton™ X-100 (Rohm & Haas Co.) are suitable and may be used in concentrations of about 0.01 to about 1.0%.

The polymer-treated membrane or support is then dried. It is preferred to air dry at ambient temperatures. However, drying may be conducted at higher or lower temperatures, if desired. Drying under vacuum conditions also will be suitable. Drying for about one to about 24 hours normally will be sufficient although longer or shorter times may be suitable for some drying conditions.

The dried support may be further treated with a humectant, if desired, to maintain the pore structure and hydrophilicity of the support material, particularly where the support material is a membrane. For example, a treated membrane may be contacted with a solution of glycerin. Most preferably, a 2-30% solution of glycerin in water is used. The humectant solution may also comprise a surfactant such as Triton X-100™, Tween-20™ or Tween-80™ at a concentration of about 0.01-2.0 percent.

In a second embodiment, the treated membrane or support comprises a dual polymeric coating which includes Darathane polymer and a second polyurethane polymer. In preparing this dual polymeric coating by a one-step process, the general procedures described above for coating with Darathane alone are followed. In this embodiment, however, the Darathane polymer solution also contains one or more of the polyurethane prepolymers described above. The polyurethane prepolymer will become polymerized and any residual functional groups will react on contact with the water in the aqueous polymer solution. The concentration of Darathane polymer in the solution is about 0.1 to about 20.0 percent, preferably about 4.0 percent. The concentration of polyurethane prepolymer in the solution is about 0.1 to about 10.0 percent, preferably about 1.0 percent.

In the two-step process for providing a dual polymer coating, the untreated membrane or support is first contacted with the polyurethane prepolymer in a volatile organic solvent. The solvent may be any of those described above with respect to the single polymer process. The concentration of prepolymer-to-solvent also will be as described above with respect to the one-step process. A surfactant may be used here as well. The support and prepolymer solution should remain in contact for at least about one minute, preferably about five to ten minutes, and most preferably at least thirty to sixty minutes. Agitation is preferred for uniformity of coating. This partially treated support is then dried as described above. The dried membrane or other support is then treated with Darathane polymer in a volatile solvent, according to the procedures described above for the one-step process. The support is dried and may be further treated with a humectant, as above.

### Characteristics of The Self-Blocked Support

The treated assay support surface of this invention is non-adsorptive for most proteins in biological samples as well as for most reagents used in immunoassays. The treated support is therefore considered to be "self-blocked" and no additional blocking step is required. Since the treated assay supports of this invention are not susceptible to nonspecific protein binding, interference from other proteins present in the test fluid is eliminated, as is possible ambiguity of test results caused by indiscriminate binding of the target protein.

Test fluids typically will contain contaminating proteins which will interfere with the assay if they become bound to the assay membrane or support. For example, if the test fluid or sample is blood or is contaminated with blood, numerous blood proteins will be present. Chief among them is hemoglobin, which is very "sticky" with respect to assay membranes typically used and which tends to mask or obliterate color indicator assay results. The self-blocked assay support of this invention substantially resists binding of hemoglobin and other proteins.

The self-blocked assay support is also quite useful in assays which employ colloidal gold as the tracer. Colloidal gold is employed in assays used to test body fluids such as urine to detect hormones, viral or bacterial antigens, or the like. Problems have been encountered in these assays since colloidal gold compounds tend to bind quite readily to conventionally blocked membranes or assay supports. The self-blocked membrane or support of this invention, on the other hand, resists nonspecific binding of colloidal gold as well as of the various other reagents typically used in diagnostic and other assays.

The self-blocked support of this invention may be used according to conventional assay methods and procedures. For example, in the embodiment utilizing a microporous membrane treated as described above, the test fluid typically will be passed through the treated membrane, preferably followed by rinsing. Next, a solution of an appropriate recognition conjugate is passed through the membrane, again preferably followed by rinsing. Finally, a substrate solution is passed through the membrane which will cause a color change in the area of the bound target protein/recognition conjugate complex. The appearance of this color change indicates the assay is positive for the target protein; lack of color change indicates the absence of the target protein in the test fluid. Using the assay support of this invention, a color change may be apparent within about 10.0 to about 20.0 seconds.

This time period and the color intensity of the indicator will depend on the nature and quality of the reagents, rather than on the membrane or support. However, the treatment procedures of this invention will provide supports on which a clear and sharp distinction is seen between positive and negative results, and between positive indicator areas and background areas of the support.

Particulate assay matrices prepared according to this invention may be used in a similar manner, that is, by sequentially passing the test and reagent solutions through a bed containing the assay matrix and observing the presence or absence of a color change. Alternatively, the particulate assay matrix may be added batchwise to the test fluid, removed by filtration or other means, then added to the reagent solutions in a similar manner. Any convenient method of contacting the assay matrix with the test fluid and reagent solutions will be suitable.

The above description is written in terms of a colorimetric assay, in which the recognition conjugate comprizes an enzyme such as horseradish peroxidase, alkaline phosphatase, or glucose oxidase which cause a visually apparent color change under positive test conditions and in the presence of a substrate specific for the enzyme. A substrate used with horseradish peroxidase is tetramethyl benzidine (TMB), with or without hydrogen peroxide. Substrates used with alkaline phosphatase are indoxyl phosphate, with or without nitro blue tetrazoleum (NBT). A substrate used with glucose oxidase is 2,2′-azino-di-(3-ethyl-benzthiazoline-G-sulfonic acid) (ABTS). Colorimetric assays also can be based on colloidal gold, rather than enzymes.

The utility of the treated supports of this invention is not limited to colorimetric assays. These assay matrices may be used with any convenient recognition and tracer or indicator system. For example, the recognition antibody may be radiolabeled for use in a radioimmunoassay. Alternatively, the indicator can be a fluorescent labelling agent that is bound to an antibody or bioaffinity agent. Examples include fluorescein isocyanate (FIC), fluoroscein isothiocyanate (FITC) and the like.

### Use Of The Treated Support In Passive Adsorption Assays

The self-blocked treated support of this invention is useful as a passive adsorption diagnostic support for detection of cell membrane-associated antigens. That is, the support may be used for this purpose without the need for an immobilized bioaffinity agent, such as an antibody or the like. Rather, the characteristics of the treated support are such that the desired cell surface antigen becomes associated with the support surface via immobilization or binding of cell membrane or fragments to the support. The cell surface antigen then may be detected on the support by ELISA or other recognition assay methods, as described above.

The treated support serves as a passive adsorption diagnostic support only for assays in which the target antigen is associated with bacterial cell membranes. Upon contact with a test sample containing whole cells or cell fragments, cell membrane becomes entrapped on or selectively bound to the treated support. For example, the treated membrane may be contacted with a lysate suspension or solution made from swabbed or scraped sample in order to bind cells or cell membrane contained therein. It is known that LPS (a component of certain cell membranes) will bind to the treated support of this invention. The treated support therefore will be suitable for use in assays for any antigen which typically is found in association with LPS or with the bacterial cell wall.

The passive adsorption support of this invention can be used to readily identify bacterial infections such as streptococcus A, chlamydia, and other bacterial diseases for which cell membrane associated antigens. A fluid test sample (i.e., urine, blood, serum, sputum, or a lysate or suspension prepared from a scrape or swab) may be passed through the membrane or over an assay support in order to bind LPS or cell membrane which may be present. The appropriate assay for detection of the desired antigen is then conducted.

The support is contacted with a recognition conjugate specific for the antigen of the disease in question, for example, an antibody-enzyme conjugate. If cells, cell membranes or LPS comprising that antigen were present in the test sample the recognition conjugate will bind to the antigen (now bound to the assay support) and a positive signal will develop on the support upon application of the appropriate substrate for the recognition conjugate. It may be desired to concentrate the test sample onto a portion of the assay support so that the positive signal will appear as a colored area against a white background. Alternatively, negative controls may be run on separate portions of the support or on separate supports.

The examples which follow are given for illustrative purposes and are not meant to limit the invention described herein. The following abbreviations have been used throughout in describing the invention.
°C - degrees Centigrade
HCG - human chorionic gonadotropin
IgG - immunoglobulin G
LPS - lipopolysaccharide(s)
M - molar
meq - milliequivalent(s)
mg - milligram(s)
ml - milliliter(s)
mm - millimeters(s)
MW - molecular weight
»l - microliter
NBT - nitro blue tetrazoleum
PBS - phosphate buffered saline
% - percent
TMB - tetramethyl benzidine

### EXAMPLE I

Microporous cellulose mixed ester (cellulose acetate/cellulose nitrate) membrane was placed in a 2.0% solution of Darathane™ WB-22 polyurethane polymer (Grace Specialty Chemicals Co., W. R. Grace & Co.-Conn.) in isopropyl alcohol under constant agitation for 30 minutes. The membrane was removed from the solution, blotted and then air dried at room temperature for four hours. The treated membrane was then immersed in an aqueous solution of 10% glycerin and 0.2% Triton™ X-100 (Sigma Chemical Co.) under constant agitation for 30 minutes, followed by air drying at room temperature for eight hours.

### EXAMPLE II

The procedures of Example I were repeated using microporous cellulose nitrate membrane.

### EXAMPLE III

Microporous nylon membranes (120 mm and 200 mm thickness) were placed in a solution of 1.0% Hypol™ 6100 polyurethane prepolymer (Grace Specialty Chemicals Co., W. R. Grace & Co.-Conn.) and 1.3% Darathane WB-22 polyurethane polymer in acetone for one hour with constant agitation. The membranes were removed from the solution and then air dried at room temperature for four hours. The treated membranes were then immersed in an aqueous solution of 10% glycerin and 0.5% Triton X-100 with constant agitation for 30 minutes. The membranes were then briefly air dried, followed by complete drying at 50°C.

### EXAMPLE IV

Microporous nylon membranes (120 mm and 200 mm thickness) were placed in a solution of 2.0% Hypol 6100 polyurethane prepolymer in acetone for one hour with constant agitation. The membranes were removed from the solution and then air dried at room temperature until completely dry. The dried membranes were then placed in a 3.0% solution of Darathane WB-22 polyurethane polymer in isopropyl alcohol with constant agitation for one hour. The membranes were removed from the solution and then air dried at room temperature for at least four hours. The treated membranes were then immersed in an aqueous solution of 10% glycerin and 0.5% Triton X-100 for one hour, followed by complete drying at room temperature or 50°C.

The experiment was repeated, substituting acetone for isopropyl alcohol in the Darathane WB-22 polyurethane polymer solution.

### EXAMPLE V

A non-woven wide-pore nylon material such as Cerex™ (James River Co.) was placed in a solution of 1.0% HYPOl 6100 polyurethane prepolymer and 2.0% Darathane WB-22 polyurethane polymer in acetone for 30 minutes with constant agitation. The material was removed from the solution and air dried for two hours at room temperature. The treated material was then immersed in an aqueous solution of 0.2% Tween-20 (Aldrich Chemical Co.) for 30 minutes with agitation, followed by complete drying at room temperature.

### EXAMPLE VI

The membranes prepared in Examples I, II, III and IV were cut into 25 mm diameter membrane discs and were placed in plastic holders over non-woven, hydrophobic polypropylene discs which in turn were placed on absorbent pads. Good contact between the discs and the absorbent pads was maintained during the assay.

A 400.0 »l positive test sample of chlamydia antigen (containing LPS) (ImmunoSearch) in extraction buffer was concentrated onto a signal zone on one area of each membrane disc. The extraction buffer was 1.0% Triton X-100 in PBS. Next, 150.0 »l of antibody-enzyme conjugate (Virostat) was added to the membrane and incubated for five minutes. The antibody was specific to chlamydia antigen; the enzyme was alkaline phosphatase. The membrane was washed with 2.0 ml wash buffer (0.002% nitro blue tetrazolium (NBT) and 0.05% Tween-20™ in PBS) and allowed to drain. Next, 150.0 »l enzyme substrate (indoxyl phosphate and NBT) was added, followed by incubation for five minutes. The membrane was washed with 2.0 ml wash buffer and incubated for five minutes. The membranes each developed a purple dot on a white background, indicating the test samples were positive for chlamydia antigen. Control membranes (extraction buffer with no chlamydia antigen) remained white. The thicker membranes, which have greater surface area, yielded darker color indications.

### EXAMPLE VII

The membranes prepared in Examples I, II, III and IV were cut into 8 mm discs and were placed in plastic holders over non-woven polyester discs which in turn were placed over several layers of absorbent material. Good contact between the discs and the absorbent was maintained during the assay.

A 300 »l positive test sample of chlamydia antigen (as in Example VI) was added to the membrane and allowed to drain. A 250 »l volume of mouse monoclonal antibody against chlamydia antigen (ImmunoSearch) was added to the membrane, followed by 250 »l goat anti-mouse antibody (Organon Teknika). Mouse IgG-horseradish peroxidase conjugate (250 »l) (Organon Teknika) was added and the membrane incubated for five minutes. Wash buffer (1.0 ml 0.05% Tween-20 in PBS) was added, followed by 250 »l substrate (tetramethyl benzidine (TMB) and hydrogen peroxide). The results were observed after five minutes. The positive test samples yielded blue discs; negative controls yielded white discs.

### EXAMPLE VIII

The membranes prepared in Examples III and IV were cut into 25 mm diameter discs and placed over glass or polypropylene supporting surfaces. To the center of each membrane was applied 2.0 »l of monoclonal antibody solution against streptococcus A, followed by air drying either at 50°C for four hours or at room temperature overnight. The membranes were then removed from the support and placed in plastic holders on absorbent pads. Good contact between the discs and the absorbend pads was maintained during the assay.

Strep A antigen (ImmunoSearch) was extracted with nitrous acid as described by Slifkin in J. Clin. Microbiol. 15:187, 1982. The neutralized antigen was added (400 »l) to each disc and incubated for one minute. Anti-strep A IgG-alkaline phosphatase conjugate (150 »l) was added and incubated for two minutes, followed by 1.0 ml wash buffer (0.05% Tween-20 in PBS). Next, 150 »l enzyme substrate solution (indoxyl phosphate and NBT) was added. The results were read after two minutes. The positive test samples yielded purple-blue discs; negative controls were white.

### EXAMPLE IX

The membranes prepared in Examples III and IV were cut into 13 mm diameter discs and placed over glass or polypropylene surfaces. To the center of each disc was applied 2.0 »l of monoclonal antibody solution against strep A, followed by air drying at room temperature overnight. The membranes were then assembled over hydrophobic discs in a device suitable for applying a vacuum.

Neutralized antigen extract (400 »l) as in Example VIII was applied to the membrane, using vacuum to aid flow when necessary. Anti-strep A IgG-horseradish peroxidase conjugate (100 »l) was added, incubated for one minute and allowed to flow through (with vacuum as necessary). The membrane was washed with 1.0 ml PBS/Tween-20 buffer. Next, 150 »l enzyme substrate solution (hydrogen peroxide and TMB). The results were read after two minutes. The positive test samples yielded blue discs; negative controls were white.

### EXAMPLE X

The membranes prepared in Examples I, II, III and IV were cut into 13 mm diameter discs and placed over glass or polypropylene supporting surfaces.

A 10 »l positive test sample of chlamydia antigen in extraction buffer (as in Example VI) was added to each disc and incubated for 15 minutes. Anti-chlamydia IgG-alkaline phosphatase enzyme conjugate (50 »l) was added and incubated for 15 minutes. The discs were drained, washed with 2.0 ml PBS/Tween-20 wash buffer over an absorbent pad and drained completely. Next, 50 »l enzyme substrate (indoxyl phosphate) was added, followed by incubation for 15 minutes. The discs were drained and the results read. The positive test samples yielded blue discs; negative controls (extraction buffer alone) were white.

### EXAMPLE XI

The membranes prepared in Examples III and IV were cut into 25 mm discs. On each disc was deposited 2.0 »l of a 1.0 mg/ml solution of rabbit anti-HCG IgG polyclonal antibody in PBS, which was dried onto the membrane at room temperature. The discs were placed in plastic holders over absorbent pads. To each disc was added a 300 »l urine sample containing HCG. Next, 100 »l anti-HCG IgG-gold conjugate was added and incubated for one minute. The discs were washed with 1.0 ml tap water, drained and read. The positive test samples yielded red dots; negative controls remained white.

### EXAMPLE XII

Cellulose Whatman paper (ET31 grade) was placed in a solution of 1.0% Hypol 6100 polyurethane prepolymer and 1.3% Darathane WB-22 polyurethane polymer in acetone for 30 minutes with constant agitation. The material was removed from the solution and dried at room temperature for two hours. The treated material was placed in an aqueous 5.0% glycerin solution containing 0.05% Tween-20 for 30 minutes. The material was then briefly air dried, followed by complete drying at 50°C.

### EXAMPLE XIII

Cellulose battery separator material (Grace Specialty Chemicals Co., W. R. Grace & Co.-Conn.) was placed in a solution of 1.0% Hypol 6100 polyurethane prepolymer and 1.3% Darathane WB-22 polyurethane polymer in acetone for 30 minutes with constant agitation. The material was removed from the solution and dried at room temperature for two hours. The treated material was placed in an aqueous 5.0% glycerin solution containing 0.05% Tween-20 for 30 minutes. The material was then briefly air dried, followed by complete drying at 50°C.

### EXAMPLE XIV

Cellulose circuit board material (Grace Specialty Chemicals Co. W. R. Grace & Co.-Conn.) was placed in a solution of 0.1% Hypol 6100 polyurethane prepolymer and 1.3% Darathane WB-22 polyurethane polymer in acetone for 30 minutes with constant agitation. The material was removed from the solution and dried at room temperature for two hours. the treated material was placed in an aqueous 5.0% glycerin solution in water containing 0.05% Tween-20 for 30 minutes. The material was then briefly air dried, followed by complete drying at 50°C.

### EXAMPLE XV

The treated materials from Examples XIII, XIV and XV were cut into 5 mm X 100 mm strips. Anti-HCG antibody was immobilized onto the strips by applying 2.0 »l of a 2.0 mg/ml solution on each strip as a dot or band, followed by drying. In small test tubes, 120 »l samples of HCG-containing urine were mixed with 35 »l assay buffer and 45 ml anti-HCG IgG-gold reagent (Ortho Diagnostics). One strip of treated material was placed in each test tube for 10 minutes, to allow the strip to absorb the liquid. The wet strips were pink in color. The strips were dried at room temperature. For positive test samples, a pink dot or band appeared against an otherwise white background. For negative controls, the dried strip was white.

The principles, preferred embodiments and modes of operation of the present invention have been described in the foregoing specification. The invention which is intended to be protected herein, however, is not to be construed as limited to the particular forms disclosed, since these are to be regarded as illustrative rather than restrictive.

## Claims

1. Use of polyurethane coated assay-supports for specific binding assays, characterized in that the assay supports are prepared by coating a support material with an aqueous-based polyurethane polymer, resulting in self-blocked assay-supports.

2. Use according to claim 1 in which said polyurethane polymer coating is prepared from a prepolymerized polyurethane polymer.

3. Use according to claims 1 or 2 in which said support material is a microporous membrane, particulate media or a non-porous assay device.

4. Use according to claims 1 to 3 in which said polymer coating further comprises a second polyurethane polymer, said second polymer having been applied to said support material as a prepolymer.

5. Use according to claim 4 in which said prepolymer is prepared by capping a polyoxalkylene polyol with a polyisocyanate compound, said prepolymer having a reaction functionality greater than 2.

6. A method for preparing a self-blocked assay support comprising:
a) selecting an assay support matrix which is a microporous membrane, particulate media or a nonporous assay device,
b) coating said support matrix with an aqueous solution comprising a polyurethane polymer and a volatile organic solvent,
c) drying the coated support matrix of step b), and
d) immobilizing a bioaffinity agent on said support.

7. The method of claim 6 in which said aqueous solution of step b) further comprises a polyurethane prepolymer.

8. A self-blocked assay support for use in assays for the testing of liquid test samples, comprising a support material having a polymeric coating of an aqueous-based polyurethane polymer thereon, and wherein a bioaffinity agent is immobilized on said support.

9. The assay support of claim 8 in which said coating is prepared from a pre-polymerized polyurethane polymer.

10. The assay support of claim 8 or 9 in which said support is a microporous membrane, particulate media or a nonporous assay device.

11. The assay support of claims 8 to 10 in which said polymer coating further comprises a second polyurethane polymer, said second polymer having been applied to said support material as a prepolymer.

12. The assay support of claim 11 in which said prepolymer is prepared by capping a polyoxalkylene polyol with a polyisocyanate compound, said prepolymer having a reaction functionality greater than 2.

## Patentansprüche

1. Verwendung von Polyurethan-beschichteten Assay-Trägern für spezifische Bindungsassays, dadurch gekennzeichnet, daß die Assay-Träger durch Beschichten eines Trägermaterials mit einem Polyurethan-Polymer auf Wasserbasis unter Bildung selbstblockierender Assay-Träger hergestellt sind.

2. Verwendung nach Anspruch 1, bei der die Polyurethan-Polymerbeschichtung aus einem präpolymerisierten Polyurethan-Polymer hergestellt ist.

3. Verwendung nach den Ansprüchen 1 oder 2, bei der das Trägermaterial eine mikroporöse Membran, partikuläre Medien oder eine nicht-poröse Assay-Vorrichtung ist.

4. Verwendung nach den Ansprüchen 1 bis 3, bei der die Polymer- Beschichtung ferner ein zweites Polyurethan-Polymer umfaßt, welches als Präpolymer auf das Trägermaterial aufgebracht wurde.

5. Verwendung nach Anspruch 4, bei der das Präpolymer durch Verkappen eines Polyoxalkylen-Polyols mit einer Polyisocyanat-Verbindung hergestellt wurde, wobei das Präpolymer eine Reaktionsfunktionalität von größer als 2 besitzt.

6. Verfahren zur Herstellung eines selbstblockierenden Assay-Trägers, das die folgenden Schritte umfaßt:
a) Auswählen einer Assay-Trägermatrix, die eine mikroporöse Membran, partikuläre Medien oder eine nicht-poröse Assay-Vorrichtung ist,
b) Beschichten dieser Trägermatrix mit einer wässrigen Lösung, die ein Polyurethan-Polymer und ein flüchtiges organisches Lösungsmittel umfaßt,
c) Trocknen der beschichteten Trägermatrix aus Stufe b) und
d) Immobilisieren eines Bioaffinitäts-Agens auf diesem Träger.

7. Verfahren nach Anspruch 6, bei dem die wässrige Lösung aus Stufe b) ferner ein Polyurethan-Präpolymer umfaßt.

8. Selbstblockierender Assay-Träger zur Verwendung in Assays zur Untersuchung flüssiger Testsubstanzen, welcher ein Trägermaterial umfaßt, auf dem sich eine polymere Beschichtung aus einem Polyurethan-Polymer auf Wasserbasis befindet, und wobei ein Bioaffinitäts-Agens auf dem Träger immobilisiert ist.

9. Assay-Träger nach Anspruch 8, bei dem die Beschichtung aus einem präpolymerisierten Polyurethan-Polymer hergestellt ist.

10. Assay-Träger nach den Ansprüchen 8 oder 9, bei dem der Träger eine mikroporöse Membran, partikuläre Medien oder eine nicht-poröse Assay-Vorrichtung ist.

11. Assay-Träger nach den Ansprüchen 8 bis 10, bei dem die Polymer-Beschichtung ferner ein zweites Polyurethan-Polymer umfaßt, welches als Präpolymer auf das Trägermaterial aufgebracht wurde.

12. Assay-Träger nach Anspruch 11, bei dem das Präpolymer durch Verkappen eines Polyoxalkylen-Polyols mit einer Polyisocyanat-Verbindung hergestellt wurde, wobei das Präpolymer eine Reaktionsfunktionalität von größer als 2 besitzt.

## Revendications

1. Utilisation de supports de test revêtus de polyuréthane pour des tests de liaison spécifique, caractérisée en ce que les supports de test sont préparés par revêtement d'un matériau de support avec un polymère polyuréthane à base aqueuse, produisant des supports de test auto-bloquants.

2. Utilisation selon la revendication 1, dans laquelle ledit revêtement de polymère polyuréthane est préparé à partir d'un polymère polyuréthane prépolymérisé.

3. Utilisation selon la revendication 1 ou 2, dans laquelle ledit matériau de support est une membrane microporeuse, un milieu particulaire ou un dispositif de test non poreux.

4. Utilisation selon les revendications 1 à 3, dans laquelle ledit revêtement de polymère comprend en outre un second polymère polyuréthane, ledit second polymère ayant été appliqué sur ledit matériau de support sous la forme d'un prépolymère.

5. Utilisation selon la revendication 4, dans laquelle ledit prépolymère est préparé par coiffage d'un polyoxyalkylène polyol avec un composé polyisocyanate, ledit prépolymère ayant un nombre de fonctions réactives supérieur à 2.

6. Méthode de préparation d'un support de test auto-bloquant, comprenant les étapes consistant à:
a) sélectionner une matrice de support de test qui est une membrane microporeuse, un milieu particulaire ou un dispositif de test non poreux,
b) revêtir ladite matrice de support avec une solution aqueuse comprenant un polymère polyuréthane et un solvant organique volatil,
c) sécher la matrice de support revêtue de l'étape b), et
d) immobiliser un agent de bioaffinité sur ledit support.

7. Méthode selon la revendication 6, dans laquelle ladite solution aqueuse de l'étape b) contient en outre un prépolymère polyuréthane.

8. Support de test auto-bloquant destiné à être utilisé dans des tests pour l'analyse d'échantillons de test liquides, comprenant un matériau de support portant un revêtement de polymère constitué d'un polymère polyuréthane à base aqueuse, et dans lequel un agent de bioaffinité est immobilisé sur ledit support.

9. Support de test selon la revendication 8, dans lequel ledit revêtement est préparé à partir d'un polymère polyuréthane prépolymérisé.

10. Support de test selon la revendication 8 ou 9, dans lequel ledit support est une membrane microporeuse, un milieu particulaire ou un dispositif de test non poreux.

11. Support de test selon les revendications 8 à 10, dans lequel ledit revêtement comprend en outre un second polymère polyuréthane, ledit second polymère ayant été appliqué sur ledit support sous la forme d'un prépolymère.

12. Support de test selon la revendication 11, dans lequel ledit prépolymère est préparé par coiffage d'un polyoxyalkylène polyol avec un composé polyisocyanate, ledit prépolymère ayant un nombre de fonctions réactives supérieur à 2.
